(19) Europäisches Patentamt
European Patent Office
Office européen des brevets

(11) Veröffentlichungsnummer : **0 345 639 B1**

# (12) EUROPÄISCHE PATENTSCHRIFT

(45) Veröffentlichungstag der Patentschrift : **09.09.92 Patentblatt 92/37**

(51) Int. Cl.[5] : **C07D 405/06,** C07D 405/14, A01N 43/653

(21) Anmeldenummer : **89109915.2**

(22) Anmeldetag : **01.06.89**

## (54) Alpha-Hydroxy-azolylethyloxirane und diese enthaltende Fungizide.

Die Akte enthält technische Angaben, die nach dem Eingang der Anmeldung eingereicht wurden und die nicht in dieserPatentschrift enthalten sind.

(30) Priorität : **04.06.88 DE 3819053**

(43) Veröffentlichungstag der Anmeldung : **13.12.89 Patentblatt 89/50**

(45) Bekanntmachung des Hinweises auf die Patenterteilung : **09.09.92 Patentblatt 92/37**

(84) Benannte Vertragsstaaten : **AT BE CH DE ES FR GB GR IT LI NL SE**

(56) Entgegenhaltungen : **EP-A- 0 251 086**

(73) Patentinhaber : **BASF Aktiengesellschaft
Carl-Bosch-Strasse 38
W-6700 Ludwigshafen (DE)**

(72) Erfinder : **Seele, Rainer, Dr.
Leiblstrasse 3
W-6701 Fussgoenheim (DE)**
Erfinder : **Kober, Reiner, Dr.
Im Schlittweg 20
W-6701 Fussgoenheim (DE)**
Erfinder : **Ammermann, Eberhard, Dr.
Sachsenstrasse 3
W-6700 Ludwigshafen (DE)**
Erfinder : **Lorenz, Gisela, Dr.
Erlenweg 13
W-6730 Neustadt (DE)**

EP 0 345 639 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

**Beschreibung**

Die vorliegende Erfindung betrifft neue Azolverbindungen, Verfahren zu ihrer Herstellung und diese enthaltende Fungizide.

Es ist bekannt, 1-[2-(2,1-Dichlorphenyl)-2-(2-propenyloxy)-ethyl]-1H-imidazol oder α-Hydroxy-azolylethyl-oxirane als Fungizide zu verwenden (GB-1 318 590, EP 251 086).

Es wurde nun gefunden, daß α-Hydroxy-azolylethyloxirane der allgemeinen Formel I

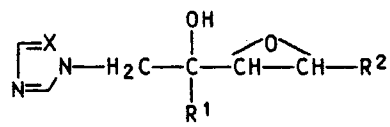

I,

in welcher $R^1$ und $R^2$ $C_3$-$C_6$-Cycloalkyl oder Phenyl bedeuten, wobei diese Reste gegebenenfalls durch Halogen oder Alkyl mit 1 bis 6 C-Atomen substituiert sind, X für CH oder N steht sowie deren für Pflanzen verträgliche Säureadditionssalze oder Metallkomplexe, eine bessere fungizide Wirkung, insbesondere gegen Getreidekrankheiten, besitzen als bekannte Azolverbindungen.

Die Verbindungen der Formel I können im Hinblick auf den Oxiranrest in cis- oder trans-Stellung vorliegen. Die trans-Verbindungen werden bevorzugt.

Die Verbindungen der Formel I enthalten asymmetrische C-Atome und können daher als Enantiomere und Diastereomere auftreten. Die Erfindung umfaßt sowohl die reinen Isomeren als auch deren Gemische. Die Gemische von Diastereomeren können nach bekannten Methoden, z.B. durch fraktionierte Kristallisation aus geeigneten Lösungsmitteln oder durch Chromatographie an Kieselgel in die Komponenten getrennt werden. Die Racemate lassen sich bei den erfindungsgemäßen Verbindungen mit üblichen Methoden, beispielsweise durch Salzbildung einer optisch aktiven Säure, Trennung der diastereomeren Salze und Freisetzung der Enantiomeren mittels einer Base, trennen. Für die Verwendung als Fungizide sind sowohl die reinen Isomeren als auch ihre bei der Herstellung entstehenden Gemische geeignet. Bevorzugt werden die Mischungen.

Die Reste $R^1$ und $R^2$ stehen beispielsweise für:

– $C_3$-$C_6$-Cycloalkyl wie Cyclopropyl, Cyclobutyl, Cyclopentyl, Cyclohexyl,

– Phenyl, wobei der aromatische Rest unsubstituiert ist oder ein oder mehrere, z.B. 1-3 Substituenten wie Halogen, z.B. Brom, Chlor oder Fluor, oder $C_1$-$C_4$-Alkyl enthält; beispielsweise seien die folgenden Reste genannt:

Phenyl, 2-Chlorphenyl, 2-Fluorphenyl, 2-Bromphenyl, 3-Chlorphenyl, 3-Bromphenyl, 3-Fluorphenyl, 4-Fluorphenyl, 4-Chlorphenyl, 4-Bromphenyl, 2,4-Dichlorphenyl, 2,3-Dichlorphenyl, 2,5-Dichlorphenyl, 2,6-Dichlorphenyl, 2-Chlor-6-fluorphenyl, 4-Ethylphenyl, 4-Isopropylphenyl, 4-tert.-Butylphenyl, 2-Chlor-4-fluorphenyl, 2-Chlor-6-methylphenyl, Cyclopropyl, Cyclobutyl, Cyclopentyl, Cyclohexyl.

Säureadditionssalze sind beispielsweise die Hydrochloride, Bromide, Sulfate, Nitrate, Phosphate, Oxalate oder Dodecylbenzolsulfonate. Die Wirksamkeit der Salze geht auf das Kation zurück, so daß es auf das Anion im allgemeinen nicht ankommt. Die erfindungsgemäßen Wirkstoffsalze werden hergestellt durch Umsetzung der Trans-α-hydroxy-azolylethyloxirane(I) mit Säuren.

Metallkomplexe der Wirkstoffe I oder ihrer Salze können mit Kupfer, Zink, Zinn, Mangan, Eisen, Kobalt oder Nickel gebildet werden, indem man die Trans-α-hydroxy-azolylethyloxirane mit entsprechenden Metallsalzen umsetzt, z.B. mit Kupfersulfat, Zinkchlorid, Zinnchlorid.

Die Verbindungen der Formel I können hergestellt werden, indem man eine Verbindung der Formel II

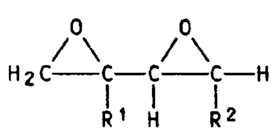

II,

in welcher $R^1$ und $R^2$ die angegebenen Bedeutungen haben, mit einer Verbindung der Formel III

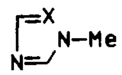

III,

in der Me ein Wasserstoffatom oder ein Metallatom bedeutet und X die angegebene Bedeutung hat, in Gegenwart einer Base zur Umsetzung bringt.

Die Reaktion erfolgt - falls Me ein Wasserstoffatom bedeutet - gegebenenfalls in Gegenwart eines Lösungs- oder Verdünnungsmittels, gegebenenfalls unter Zusatz eines Reaktionsbeschleunigers bei Temperaturen zwischen 10 und 150, insbesondere 20 und 120°C. Zu den bevorzugten Lösungs- und Verdünnungsmitteln gehören Ketone wie Aceton, Methylethylketon oder Cyclohexanon, Nitrile wie Acetonitril oder Propionitril, Alkohole wie Methanol, Ethanol, iso-Propanol, n-Butanol oder Glycol, Ester wie Essigsäureethylester, Essigsäuremethylester oder Essigsäurebutylester, Ether wie Tetrahydrofuran, Diethylether, Dimethoxyethan, Dioxan oder Diisopropylether, Amide wie Dimethylformamid, Dimethylacetamid oder N-Methylpyrrolidon, ferner Dimethylsulfoxid, Sulfolan oder entsprechende Gemische.

Geeignete Basen, die gegebenenfalls auch als säurebindende Mittel bei der Reaktion verwendet werden können, sind beispielsweise Alkalihydroxid wie Natrium-, Kaliumhydroxid, Carbonate wie Lithium-, Natrium- oder Caesiumcarbonat oder Natrium, Kalium- oder Caesiumhydrogencarbonat, Pyridin oder 4-Dimethylaminopyridin. Es können aber auch andere übliche Basen verwendet werden.

Als Reaktionsbeschleuniger kommen vorzugsweise Metallhalogenide wie Natriumiodid, oder Kaliumiodid, quarternäre Ammoniumsalze wie Tetrabutylammoniumchlorid, -bromid, iodid oder -hydrogensulfat, Benzyltriethylammoniumchlorid oder -bromid oder Kronenether wie 12-Krone-4, 15-Krone-5, 18-Krone-6, Dibenzo-18-krone-6 oder Dicyclohexano-18-krone-6 in Frage.

Die Umsetzung wird im allgemeinen drucklos oder unter Druck, kontinuierlich oder diskontinuierlich nach den dafür üblichen Techniken durchgeführt.

Ist Me ein Metallatom wird die Reaktion gegebenenfalls in Gegenwart eines Lösungs- oder Verdünnungsmittels und gegebenenfalls unter Zusatz einer starken anorganischen oder organischen Base bei Temperaturen zwischen -10 und 120°C durchgeführt. Zu den bevorzugten Lösungs- und Verdünnungsmitteln gehören Amide wie Dimethylformamid, Diethylformamid, Dimethylacetamid, Diethylacetamid, N-Methylpyrrolidon, Hexamethyl-phosphortriamid, Sulfoxide wie Dimethylsulfoxid und schließlich Sulfolan.

Geeignete Basen, die gegebenenfalls auch als säurebindende Mittel bei der Reaktion verwendet werden können, sind beispielsweise Alkalihydride wie Lithium-, Natrium- und Kaliumhydrid, Alkaliamide wie Natrium- und Kaliumamid, ferner Natrium- oder Kalium-tert.-butoxid, Lithium-, Natrium- oder Kalium-triphenylmethyl und Naphthalinlithium, -natrium oder -kalium.

Die Reaktion wird im allgemeinen zwischen 0 und 100°C, vorzugsweise bei 20 bis 80°C durchgeführt. Bei Anwesenheit eines Lösungsmittels wird zweckmäßigerweise beim Siedepunkt des jeweiligen Lösungsmittels umgesetzt.

Zur Herstellung der erfindungsgemäßen Verbindungen I nach den vorstehend beschriebenen Methoden setzt man die Ausgangsstoffe üblicherweise im stöchiometrischen Verhältnis ein. Ein Überschuß der einen oder anderen Komponente (bis zu 10 %) kann gegebenenfalls von Vorteil sein.

Man setzt normalerweise mindestens äquivalente Mengen der Base, bezogen auf III zu, kann diese aber auch im Überschuß verwenden.

Die neuen Ausgangsverbindungen II lassen sich nach bekannten Methoden in einfacher Weise aus den Oxiranen der Formel IV

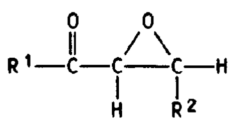

IV

herstellen, z.B. durch Umsetzung mit Trimethylsulfonium-methylsulfat (vgl. Corey, Chaykovsky, J. Am. Chem. Soc. 1962, 64, 3782ff).

Die Epoxide IV lassen sich aus den Olefinen V nach bekannten Verfahren herstellen. Dazu oxidiert man die Olefine V

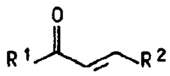

V

mit Peroxycarbonsäuren wie Perbenzoesäure, 3-Chlorperbenzoesäure, 4-Nitroperbenzoesäure, Monoperphthalsäure, Peressigsäure, Perpropionsäure, Permaleinsäure, Monoperbernsteinsäure, Perpelargonsäure oder Trifluorperessigsäure in indifferenten Lösungsmitteln, vorzugsweise chlorierten Kohlenwasserstoffen, z.B. Methylenchlorid, Chloroform, Tetrachlorkohlenstoff, Dichlorethan, oder gegebenenfalls auch in Essigsäure, Essigester, Aceton oder Dimethylformamid, gegebenenfalls in Gegenwart eines Puffers wie Natriumacetat, Natriumcarbonat, Dinatriumhydrogenphosphat, Triton B. Man arbeitet zwischen 10 und 100°C und katalysiert die Reaktion gegebenenfalls z.B. mit Iod, Natriumwolframat oder Licht. Zur Oxidation eignen sich auch alkalische Lösungen von Wasserstoffperoxid (ca. 30 %) in Methanol, Ethanol, Aceton oder Acetonitril bei 25 bis 30°C sowie Alkylhydroperoxide, z.B. tert.-Butylhydroperoxid, unter Zusatz eines Katalysators, z.B. Natriumwolframat, Perwolframsäure, Molybdänhexacarbonyl oder Vanadylacetylacetonat. Die genannten Oxidationsmittel lassen sich zum Teil in situ erzeugen.

Die entsprechenden cis-konfigurierten Verbindungen können durch Isomerisierung der trans-Verbindungen in an sich bekannter Weise, wie z.B. in EP 240 216 beschrieben, hergestellt werden.

Die Verbindungen V lassen sich entsprechend allgemein bekannten Verfahren zur Olefinsynthese (Houben-Weyl-Müller, Methoden der organischen Chemie, Georg Thieme Verlag, Stuttgart, 1972, Bd V, 1b) herstellen.

Die folgenden Beispiele erläutern die Herstellung der Wirkstoffe.

I. Herstellung der Ausgangsstoffe

Vorschrift A

98,2 g E-Phenyl-2-chlorphenylstyrylketon werden in 300 ml Methanol gelöst und 8 ml Natronlauge (50 %ig) zugesetzt. Die Reaktionslösung wird bei 0°C gerührt, während 30,3 g Wasserstoffperoxid (ca. 50 %ig) langsam zugetropft werden, wobei die Innentemperatur von 30°C nicht überschritten wird. Anschließend wird das Reaktionsgemisch sechs Stunden bei Raumtemperatur (20°C) gerührt und aufgearbeitet, indem man der Lösung 50 ml Wasser zusetzt und den entstandenen Niederschlag absaugt. Man erhält 94,6 g (90 %) 2-Benzoyl-3-(2-chlorphenyl)-oxiran, Fp.: 132°C.

Vorschrift B

Zu einer Lösung von 39 g 2-Benzoyl-3-(2-chlorphenyl)-oxiran in 250 ml Methylenchlorid werden 28 g Trimethylsulfoniummethylsulfat und 70 ml Natronlauge (50 %ig) gegeben. Nachdem das Reaktionsgemisch 12 bis 15 Stunden bei Raumtemperatur gehalten wurde, wird der Lösung 200 ml Wasser zugesetzt und die organische Phase abgetrennt. Die verbleibende wässrige Phase wird mit Methylenchlorid extrahiert und die gesammelten organischen Phasen mit Wasser gewaschen. Nach Trocknen über Natriumsulfat und Einengen der organischen Phase werden 34 g (83 %) 2-[(2-Phenyl)-oxiran-2-yl]-3-(2-chlorphenyl)-oxiran erhalten, das ohne weitere Reinigung mit Triazol gemäß dem folgenden Beispiel umgesetzt wird.

II. Herstellung der Endprodukte

Beispiel 1

Eine Lösung von 3,7 g Triazol in 50 ml N-Methylpyrrolidon wird mit 9,4 g Kaliumcarbonat versetzt und anschließend bei Raumtemperatur 11,2 g 2-[(2-Phenyl)-oxiran-2-yl]-3-(2-chlorphenyl)-oxiran, das in 50 ml N-Methylpyrrolidon gelöst ist, langsam zugetropft. Nachdem das Reaktionsgemisch 10 bis 18 Stunden bei Raumtemperatur gerührt wurde, wird der Lösung 100 ml Wasser zugesetzt und das Gemisch mehrmals mit Methyl-tert.-butylether ausgeschüttelt. Die isolierte organische Phase wird mit Wasser gewaschen, über Natriumsulfat getrocknet und eingeengt. Durch Kristallisation des verbleibenden Rückstandes aus Methyl-tert.-butylether/n-Hexan erhält man 8,1 g (58 %) trans-2-[1-Hydroxy-1-phenyl-2-(1,2,4-triazol-1-yl)-ethyl]-3-(2-chlorphenyl)-oxiran mit einem Schmelzpunkt von 180 bis 182°C (Verbindung Nr. 1).

Entsprechend Beispiel 1 können die in der Tabelle aufgeführten verbindungen hergestellt werden.

Tabelle: Trans-α-hydroxy-azolylethyloxirane

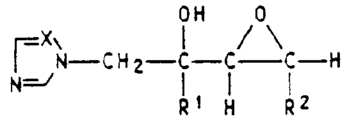

| Beispiel | $R^1$ | $R^2$ | X | Schmp./IR | Bemerkungen |
|---|---|---|---|---|---|
| 1 | $C_6H_5$ | $2-Cl-C_6H_4$ | N | 180-182°C | Enantiomerengemisch |
| 2 | $C_6H_5$ | $2-Cl-C_6H_4$ | CH | - | - |
| 3 | $C_6H_5$ | $4-Cl-C_6H_4$ | N | - | - |
| 4 | $C_6H_5$ | $4-F-C_6H_4$ | N | - | - |
| 5 | $C_6H_5$ | $2,4-Cl_2-C_6H_3$ | N | - | - |
| 6 | $C_6H_5$ | $2-Cl-4-F-C_6H_3$ | N | - | - |
| 10 | $C_6H_5$ | 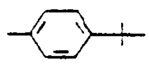 | N | 179-180°C | Enantiomerengemisch |
| 11 | $C_6H_5$ | Cyclohexyl | N | - | - |
| 12 | $C_6H_5$ | Cyclohexyl | CH | - | - |
| 14 | $4-Cl-C_6H_4$ | $C_6H_5$ | N | 121°C | $D_1:D_2=2:1$ |
| 15 | $4-Cl-C_6H_4$ | $2-Cl-C_6H_4$ | N | 208-210°C | Enantiomerengemisch |
| 16 | $4-Cl-C_6H_4$ | $2-Cl-C_6H_4$ | CH | 170°C | Enantiomerengemisch |
| 17 | $4-Cl-C_6H_4$ | $4-Cl-C_6H_4$ | N | - | - |
| 18 | $4-Cl-C_6H_4$ | $4-Cl-C_6H_4$ | CH | - | - |
| 19 | $4-Cl-C_6H_4$ | $2-F-C_6H_4$ | N | 125-127°C | $D_1:D_2=1,2:1$ |
| 20 | $4-Cl-C_6H_4$ | $4-F-C_6H_4$ | N | Harz | $D_1:D_2=1,2:1$ |
| 21 | $4-Cl-C_6H_4$ | $4-F-C_6H_4$ | CH | - | - |
| 22 | $4-Cl-C_6H_4$ | $2,4-Cl_2-C_6H_3$ | N | - | - |
| 23 | $4-Cl-C_6H_4$ | $2-Cl-4-F-C_6H_3$ | N | - | - |
| 24 | $4-Cl-C_6H_4$ | Cyclohexyl | N | - | - |
| 25 | $4-Cl-C_6H_4$ | Cyclohexyl | CH | - | - |

$D_1$ = Diastereomer 1 $D_2$ = Diastereomer 2

| Beispiel | $R^1$ | $R^2$ | X | Schmp./IR | Bemerkungen |
|---|---|---|---|---|---|
| 27 | $4-F-C_6H_4$ | $2-Cl-C_6H_4$ | N | 192-193°C | Enantiomerengemisch |
| 28 | $4-F-C_6H_4$ | $2-Cl-C_6H_4$ | CH | 150°C | Enantiomerengemisch |
| 29 | $4-F-C_6H_4$ | $4-Cl-C_6H_4$ | N | - | - |
| 30 | $4-F-C_6H_4$ | $4-Cl-C_6H_4$ | CH | - | - |
| 31 | $4-F-C_6H_4$ | $2-F-C_6H_4$ | N | - | - |
| 32 | $4-F-C_6H_4$ | $4-F-C_6H_4$ | N | - | - |
| 33 | $4-F-C_6H_4$ | $2,4-Cl_2-C_6H_3$ | N | - | - |
| 35 | $4-F-C_6H_4$ | Cyclohexyl | N | - | - |
| 41 | Cyclopropyl | $2-Cl-C_6H_4$ | N | Harz | $D_1:D_2=1,3:1$ |
| 42 | Cyclopropyl | $2-Cl-C_6H_4$ | CH | - | - |
| 43 | Cyclopropyl | $4-Cl-C_6H_4$ | N | 170-172°C | Enantiomerengemisch |
| 44 | Cyclopropyl | $2-F-C_6H_4$ | N | - | - |
| 45 | Cyclopropyl | $4-F-C_6H_4$ | N | 130-133°C | Enantiomerengemisch |
| 46 | Cyclopropyl | $2,4-Cl_2-C_6H_3$ | CH | - | - |
| 47 | Cyclopropyl | Cyclohexyl | N | - | - |
| 49 | Cyclohexyl | $2-Cl-C_6H_4$ | N | 238-246°C | Enantiomerengemisch |
| 50 | Cyclohexyl | $2-Cl-C_6H_4$ | CH | - | - |
| 51 | Cyclohexyl | $4-Cl-C_6H_4$ | N | - | - |
| 52 | Cyclohexyl | $2-F-C_6H_4$ | N | - | - |
| 53 | Cyclohexyl | $4-F-C_6H_4$ | N | 160°C | Enantiomerengemisch |
| 54 | Cyclohexyl | Cyclohexyl | N | - | - |

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Rasen, Baumwolle, Soja, Kaffee, Zuckerrohr, Obst und Zierpflanzen im Gartenbau, Weinbau sowie Gemüse - wie Gurken, Bohnen und Kürbisgewächse -.

Die neuen Verbindungen sind insbesondere geeignet zur Bekämpfung folgender Pflanzenkrankheiten:

Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,

Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff oder mehr je ha. Die neuen Verbindungen können auch im Materialschutz eingesetzt werden, z.B. gegen Paecilomyces variotii.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

Beispiele für solche Zubereitungen sind:

I. Man vermischt 90 Gew.-Teile der Verbindung Nr. 15 mit 10 Gew.-Teilen N-Methyl-α-pyrrolidon und erhält eine Lösung, die zur Anwendung in Form kleinster Tropfen geeignet ist.

II. 20 Gew.-Teile der Verbindung Nr. 19 werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure und 5 Gew.-Teilen des Anlagerungsproduktes und 40 Mol Ethylenoxid an 1 Mol Ricinusöl besteht. Durch Ausgießen und feines Verteilen der Lösung in Wasser erhält man eine wäßrige Dispersion.

III. 20 Gew.-Teile der Verbindung Nr. 49 werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gew.-Teile der Verbindung Nr. 53 werden in einer Mischung gelöst, die aus 25 Gew.-Teilen Cyclohexanol, 65 Gew.-Teilen einer Mineralölfraktion vom Siedepunkt 210 bis 280°C und 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gew.-Teile der Verbindung Nr. 15 werden mit 3 Gew.-Teilen des Natriumsalzes der Diisobutylnaphthalin-α-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.

VI. 3 Gew.-Teile der Verbindung Nr. 19 werden mit 97 Gew.-Teilen feinteiligem Kaolin innig vermischt. Man erhält auf diese Weise ein Stäubemittel, das 3 Gew.% des Wirkstoffs enthält.

VII. 30 Gew.-Teile der Verbindung Nr. 49 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gew.-Teile der Verbindung Nr. 53 werden mit 10 Gew.-Teilen Natriumsalz eines Phenolsulfonsäure-harnstoff-formaldehyd-Kondensates, 2 Gew.-Teilen Kieselgel und 48 Gew.-Teilen Wasser innig vermischt. Man erhält eine stabile wäßrige Dispersion. Durch Verdünnen mit Wasser erhält man eine wäßrige Dispersion.

IX. 20 Gew.-Teile der Verbindung Nr. 15 werden mit 2 Gew.-Teilen Calciumsalz der Dodecylbenzolsulfonsäure, 8 Gew.-Teilen Fettalkoholpolyglykolether, 2 Gew.-Teilen Natriumsalz eines Phenolsulfonsäure-harnstoff-formaldehyd-Kondensats und 68 Gew.-Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Anwendungsbeispiele

Als Vergleichswirkstoff wurde 1-[2-(2,4-Dichlorphenyl)-2-(2-propenyloxy)-ethyl]-1H-imidazol (A) - bekannt aus GB-PS 1 318 590 - benutzt.

Anwendungsbeispiel 1

Wirksamkeit gegen Weizenbraunrost

Blätter von in Töpfen gewachsenen Weizensämlingen der Sorte "Frühgold" wurden mit Sporen des Braunrostes (Puccinia recondita) bestäubt. Danach wurden die Töpfe für 24 Stunden bei 20 bis 22°C in einer Kammer mit hoher Luftfeuchtigkeit (94 bis 95 %) gestellt. Während dieser Zeit keimten die Sporen aus und die Keimschläuche drangen in das Blattgewebe ein. Die infizierten Pflanzen wurden anschließend mit wäßrigen Spritzbrühen, die 80 % Wirkstoff und 20 % Emulgiermittel in der Trockensubstanz enthielten, tropfnaß gespritzt. Nach dem Antrocknen des Spritzbelages wurden die Versuchspflanzen im Gewächshaus bei Temperaturen zwischen 20 und 22°C und 65 bis 70 % relativer Luftfeuchte aufgestellt. Nach 8 Tagen wurde das Ausmaß der Rostpilzentwicklung auf den Blättern ermittelt.

Das Ergebnis zeigt, daß die Wirkstoffe 15, 19, 49 und 53 bei der Anwendung als 0,025 %ige ( Gew.%) Spritzbrühen eine bessere fungizide Wirkung zeigen (97 %) als der bekannte Vergleichswirkstoff A (80 %).

## Patentansprüche

1. α-Hydroxy-azolylethyloxirane der allgemeinen Formel I

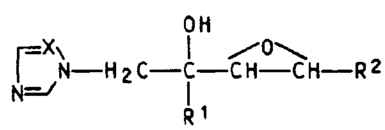

I,

in welcher $R^1$ und $R^2$ $C_3$-$C_6$-Cycloalkyl oder Phenyl bedeuten, wobei diese Reste gegebenenfalls durch Halogen oder Alkyl mit 1 bis 4 C-Atomen substituiert sind, X für CH oder N steht sowie deren für Pflanzen verträgliche Säureadditionssalze oder Metallkomplexe.

2. Verbindungen der Formel I gemäß Anspruch 1, die am Oxiranring trans-konfiguriert vorliegen.

3. Verfahren zur Herstellung der trans-α-Hydroxy-azolylethyloxirane der Formel I gemäß Anspruch 2, dadurch gekennzeichnet, daß man eine Verbindung der Formel II

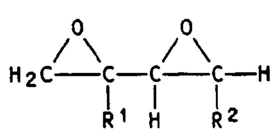

II,

in welcher $R^1$ und $R^2$ die angegebenen Bedeutungen haben mit einer Verbindung der Formel III

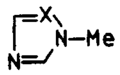

III,

in der Me ein Wasserstoffatom oder ein Metallatom bedeutet und X die angegebene Bedeutung hat, in Gegenwart einer ßase zur Umsetzung bringt und die so erhaltenen Verbindungen gegebenenfalls in ihre für Pflanzen verträglichen Salze oder Metallkomplexe überführt.

4. Fungizides Mittel, enthaltend eine fungizid wirksame Menge eines α-Hydroxy-azolylethyloxirans der allgemeinen Formel I

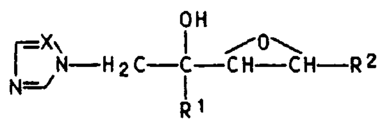

I,

in welcher $R^1$ und $R^2$ $C_3$-$C_6$-Cycloalkyl, Phenyl bedeuten, wobei die Reste gegebenenfalls durch Halogen, Alkyl mit l bis 4 C-Atomen substituiert sind, X für CH oder N steht oder dessen für Pflanzen verträglichen Säureadditionssalzes oder Metallkomplexes und einen inerten Zusatzstoff.

5. Verfahren zur Bekämpfung von Pilzen, dadurch gekennzeichnet, daß man eine fungizid wirksame Menge eines α-Hydroxy-azolylethyloxirans der allgemeinen Formel I

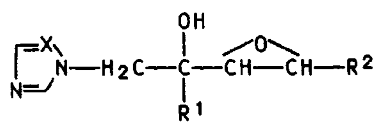

I,

in welcher $R^1$ und $R^2$ $C_3$-$C_6$-Cycloalkyl, Phenyl bedeuten, wobei diese Reste gegebenenfalls durch Halogen, Alkyl mit l bis 4 C-Atomen substituiert sind, X für CH oder N steht oder dessen für Pflanzen verträglichen Säureadditionssalzes oder Metallkomplexes auf die Pilze oder auf durch Pilzbefall bedrohte Materialien, Flächen, Pflanzen oder Saatgüter einwirken läßt.

6. Verbindung der Formel I gemäß Anspruch 1, in der $R^1$ 4-Chlorphenyl und $R^2$ 2-Chlorphenyl bedeutet und X die Bedeutung N hat, in der trans-Stellung.

7. Verbindung der Formel I gemäß Anspruch 1, in der $R^1$ 4-Chlorphenyl und $R^2$ 2-Fluorphenyl bedeutet und X die Bedeutung N hat, in der trans-Stellung.

8. Verbindung der Formel I gemäß Anspruch 1, in der $R^1$ Cyclohexyl und $R^2$ 2-Chlorphenyl bedeutet und X die Bedeutung N hat, in der trans-Stellung.

9. Verbindung der Formel I gemäß Anspruch 1, in der $R^1$ Cyclohexyl und $R^2$ 4-Fluorphenyl bedeutet und X die Bedeutung N hat, in der trans-Stellung.

## Claims

1. An α-hydroxyazolylethyloxirane of the general formula I

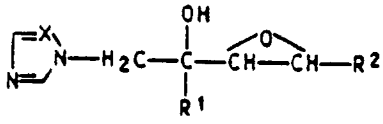

I,

where $R^1$ and $R^2$ are each $C_3$-$C_6$-cycloalkyl or phenyl, these radicals being unsubstituted or substituted by halogen or alkyl of 1 to 4 carbon atoms, X is CH or N, and plant-tolerated acid addition salts or metal complexes thereof.

2. A compound of the formula I as claimed in claim 1 and having a trans configuration with respect to the oxirane ring.

3. A process for the preparation of a trans-α-hydroxyazolylethyloxirane of the formula I as claimed in claim 2, wherein a compound of the formula II

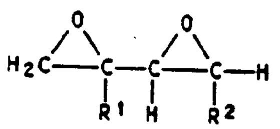

II,

where $R^1$ and $R^2$ have the stated meanings, is reacted with a compound of the formula III

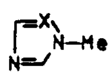

III,

where Me is hydrogen or a metal atom and X has the stated meanings, in the presence of a base, and the compound thus obtained is, if required, converted into its plant-tolerated salts or metal complexes.

4. A fungicidal agent containing a fungicidally effective amount of an α-hydroxyazolylethyloxirane of the general formula I

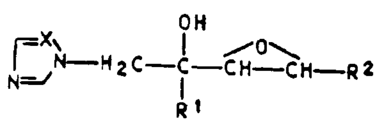

I,

where $R^1$ and $R^2$ are each $C_3$-$C_6$-cycloalkyl or phenyl, the radicals being unsubstituted or substituted by halogen or alkyl of 1 to 4 carbon atoms, X is CH or N, or a plant-tolerated acid addition salt or metal complex thereof, and an inert additive.

5. A process for controlling fungi, wherein a fungicidally effective amount of an α-hydroxyazolylethyloxirane of the general formula I

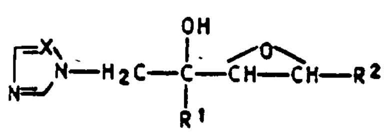

I.

where $R^1$ and $R^2$ are each $C_3$-$C_6$-cycloalkyl or phenyl, these radicals being unsubstituted or substituted by halogen or alkyl of 1 to 4 carbon atoms, X is CH or N, or a plant-tolerated acid addition salt or metal complex thereof, is allowed to act on the fungi or on the materials, areas, plants or seed threatened by fungus.

6. A compound of the formula I as claimed in claim 1, where $R^1$ is 4-chlorophenyl, $R^2$ is 2-chlorophenyl and X is N, in the trans configuration.

7. A compound of the formula I as claimed in claim 1, where $R^1$ is 4-chlorophenyl, $R^2$ is 2-fluorophenyl and X is N, in the trans configuration.

8. A compound of the formula I as claimed in claim 1, where $R^1$ is cyclohexyl, $R^2$ is 2-chlorophenyl and X is

N, in the trans configuration.

9. A compound of the formula I as claimed in claim 1, where $R^1$ is cyclohexyl, $R^2$ is 4-fluorophenyl and X is N, in the trans configuration.

## Revendications

1. Alpha-hydroxy-azolyléthyloxirannes de formule générale I

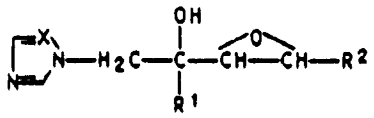

I,

dans laquelle $R^1$ et $R^2$ représentent des groupes cycloalkyle en C3-C6 ou phényle, qui peuvent le cas échéant être substitués par des halogènes ou des groupes alkyle en C1-C4, X représente CH ou N, et leurs sels formés par addition avec des acides ou complexes métalliques tolérés par les végétaux.

2. Composés de formule I de la revendication 1, en configuration trans sur le cycle oxyranne.

3. Procédé de préparation des trans-alpha-hydroxy-azolyléthyloxirannes de formule I de la revendication 2, caractérisé en ce que l'on fait réagir un composé de formule II

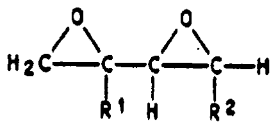

II,

dans laquelle $R^1$ et $R^2$ ont les significations indiquées ci-dessus, avec un composé de formule III

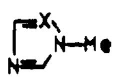

III,

dans laquelle Me représente un atome d'hydrogène ou un atome métallique et X a les significations indiquées ci-dessus, en présence d'une base, et le cas échéant on convertit les composés ainsi obtenus en leurs sels ou complexes métalliques tolérés par les végétaux.

4. Produit fongicide contenant une quantité fongicide efficace d'un alpha-hydroxy-azolyléthyloxiranne de formule générale I

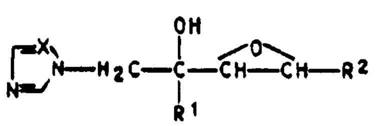

I,

dans laquelle $R^1$ et $R^2$ représentent des groupes cycloalkyle en C3-C6 ou phényle, ces groupes pouvant éventuellement être substitués par des halogènes ou des groupes alkyle en C1-C4, X représente CH ou N, ou de l'un de ses sels ou complexes métalliques tolérés par les végétaux, et un additif inerte.

5. Procédé pour combattre les mycètes, caractérisé en ce qeu l'on fait agir une quantité fongicide efficace d'un alpha-hydroxy-azolyléthyloxiranne de formule générale I

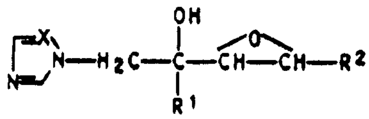

I,

dans laquelle $R^1$ et $R^2$ représentent des groupes cycloalkyle en C3-C6, phényle, ces groupes pouvant le cas échéant être substitués par des halogènes, des groupes alkyle en C1-C4, X représente CH ou N, ou de l'un de ses sels formés par addition avec un acide ou complexes métalliques tolérés par les végétaux, sur les mycètes ou sur les matériaux, surfaces, végétaux ou semences menacés d'une attaque par les mycètes.

6. Composé de formule I de la revendication 1, dans laquelle $R^1$ représente un groupe 4-chlorophényle et $R^2$ un groupe 2-chlorophényle, et X représente N, en configuration trans.

7. Composé de formule I de la revendication 1, dans laquelle $R^1$ représente un groupe 4-chlorophényle et $R^2$ un groupe 2-fluorophényle et X représente N, en configuration trans.

8. Composé de formule I de la revendication 1, dans laquelle $R^1$ représente un groupe cyclohexyle et $R^2$ un groupe 2-chlorophényle et X représente N, en configuration trans.

9. Composé de formule I de la revendication 1, dans laquelle $R^1$ représente un groupe cyclohexyle et $R^2$ un groupe 4-fluorophényle et X représente N, en configuration trans.